# EUROPEAN PATENT APPLICATION

(11) **EP 3 981 255 A1**
(43) Date of publication of application: **13.04.2022**
(21) Application number: 20817852.5
(22) Date of filing: 05.06.2020
(51) Int. Cl.: A23C 9/152, A61P 1/04, A61P 3/02, A61P 31/00, A61P 31/14, A61P 43/00, A23L 33/125, A23L 33/19, A61K 38/40, A61K 31/702

(54) **NUTRITIONAL COMPOSITION**

(30) Priority: 05.06.2019 JP 2019105506
(71) Applicant: Morinaga Milk Industry Co., Ltd., Minato-ku Tokyo 108-8384 (JP)
(72) Inventor: WADA, Yasuaki, Zama-shi, Kanagawa 252-8583 (JP); EHARA, Tatsuya, Zama-shi, Kanagawa 252-8583 (JP)
(74) Representative: Vossius & Partner Patentanwälte Rechtsanwälte mbB
(86) International application number: PCT/JP2020/022294
(87) International publication number: WO 2020/246585

(57) **Abstract**

An object of the present invention is to provide a technique for preventing infection of pathogenic viruses, which cause infectious diseases in animals or humans. A nutritional composition containing lactoferrin and/or lactoferrin hydrolysate, and human milk oligosaccharide is provided. One or more human milk oligosaccharides are preferably 2'-fucosyllactose, 3-fucosyllactose, 3'-sialyllactose, and 6'-sialyllactose. The nutritional compositions of the present invention can be used for preventing infection, especially for preventing viral infection.

## Description

### Technical Field

The present invention relates to a nutritional composition containing lactoferrin and/or a lactoferrin hydrolysate, and a human milk oligosaccharide.

### Background Art

In nature, there are various microorganisms that cause infectious diseases in a human or animal. Those microorganisms are classified into bacteria, mycoplasmas, rickettsias, chlamydias, fungi, protozoas, and viruses. The genetic material of viruses is deoxyribonucleic acid (DNA) or ribonucleic acid (RNA), and viruses can proliferate only by parasitism in host cells, whereas bacteria and other microorganisms have energy metabolism systems and heterotrophically proliferate (for example, Non-patent document 1).

Influenza virus infectious diseases are prevalent in winter, and infants, young children, elderly persons, and patients with underlying diseases such as a malignant tumor, heart disease, and cerebrovascular disease constitute a high-risk group for influenza virus infection, and often develop life-threatening critical infections.

Although antibiotics have been developed and effective treatments are performed especially for bacterial infectious diseases among microbial infectious diseases, such antibiotics are not effective for treating viral infectious diseases. The present situation is that development of antiviral agents as therapeutic drugs for viral infectious diseases is not progressing so much even now in contrast to the rapid progress of chemotherapies using antibiotics.

The antiviral agents developed so far include aciclovir, azidothymidine, and ribavirin, which are nucleic acid analogues that target replication of viral nucleic acid, zanamivir (4-guanidinoneuraminic acid), which is a neuraminidase inhibitor, indinavir, which is a protease inhibitor, and so forth. Although these antiviral agents as drugs show potent efficacy, side reactions caused by administration of these cause serious clinical problems.

Because of such a situation, there has been continued search for active ingredients showing antiviral action, which are safe, show few side reactions, and can be ingested over a long period of time.

Lactoferrin (henceforth also abbreviated as LF) is a milk protein, and has been reported to have various biological functions such as antibacterial activity, antiviral activity, action for controlling iron absorption from intestinal tract, cell proliferating action, and immunoregulating action (Non-patent document 2), and use thereof as an influenza virus infection-preventing agent has already been proposed (Patent document 1). It has also been reported that lactoferrin decomposition products (peptides derived from lactoferrin) also show an antiviral activity against influenza viruses and RS viruses (Patent document 2).

It has also been reported that human milk oligosaccharide (henceforth also abbreviated as HMO), which is a general term for referring to various oligosaccharides contained in human colostrums, can prevent viral infections (Patent document 3).

### Prior art references

### Patent documents

Patent document 1: Japanese Patent No. 3061376
Patent document 2: Japanese Patent Laid-open (KOKAI) No. 2005-272438
Patent document 3: Japanese Patent Laid-open (KOHYO) No. 2017-515455

### Non-patent documents

Non-patent document 1: Medical Virology, 2nd revised edition, Ed. by Osato Toyoro, Nankodo, 2000, pp.15-24
Non-patent document 2: B. Lonnerdal et al., Ann. Rev. Nutr., Vol. 15, 1995, pp.93-110

### Summary of the Invention

### Object to be Achieved by the Invention

An object of the present invention is to provide a technique for preventing infection of pathogenic viruses and so forth, which causes infectious diseases in animals and human.

### Means for Achieving the Object

The inventors of the present invention conducted various research in order to achieve the aforementioned object, and as a result, they found that a nutritional composition containing lactoferrin and/or a lactoferrin hydrolysate, and a human milk oligosaccharide has an infection-preventing effect, and accomplished the present invention.

The present invention thus provides a nutritional composition containing lactoferrin and/or a lactoferrin hydrolysate, and a human milk oligosaccharide.

According to the present invention, the mass ratio of the human milk oligosaccharide to the lactoferrin and/or lactoferrin hydrolysate is preferably not lower than 1/100 and not higher than 100.

According to the present invention, the content of the lactoferrin and/or lactoferrin hydrolysate based on the whole nutritional composition is preferably 0.001 to 10.0 mass %.

According to the present invention, the content of the human milk oligosaccharide based on the entire nutritional composition is preferably 0.001 to 10.0 mass %.

According to the present invention, the human milk oligosaccharide preferably contains one or more of 2'-fucosyllactose, 3-fucosyllactose, 3'-sialyllactose, and 6'-sialyllactose.

The nutritional composition of the present invention can be preferably used for preventing infection, and can be more preferably used for preventing gastrointestinal tract infection.

The nutritional composition of the present invention is preferably in the form of a powdered formula or a liquid formula.

The nutritional composition of the present invention is preferably administered to infants, young children, or elderly persons.

### Brief Description of the Drawings

[Fig. 1] A graph showing infection-suppressing ratio for rotavirus in cells to which lactoferrin and/or 2'-fucosyllactose was added.
[Fig. 2] A graph showing infection-suppressing ratio for rotavirus in cells to which lactoferrin and/or 3-fucosyllactose was added.
[Fig. 3] A graph showing infection-suppressing ratio for rotavirus in cells to which lactoferrin and/or 3-fucosyllactose was added.
[Fig. 4] A graph showing infection-suppressing ratio for rotavirus in cells to which lactoferrin and/or 3'-sialyllactose was added.
[Fig. 5] A graph showing infection-suppressing ratio for rotavirus in cells to which lactoferrin and/or 6'-sialyllactose was added.
[Fig. 6] A graph showing infection-suppressing ratio for rotavirus in cells to which lactoferrin and/or 6'-sialyllactose was added.
[Fig. 7] A graph showing infection-suppressing ratio for rotavirus in cells to which lactoferrin and/or human milk oligosaccharides (mixture) were added.

### Effect of the Invention

According to the present invention, a nutritional composition suitable for use in prevention of infection is provided. This nutritional composition can be contained in foods, drinks, and drugs as an additive or the like. Further, use of the nutritional composition of the present invention itself as a food or drink is expected to be useful for health maintenance of especially infants, young children, and elderly persons.

### Modes for Carrying out the Invention

Hereafter, the present invention will be explained in detail. However, the present invention is not limited to the following embodiments but can be freely changed within the scope of the present invention.

The nutritional composition of the present invention contains lactoferrin and/or a lactoferrin hydrolysate, and a human milk oligosaccharide.

Lactoferrin is an iron-binding glycoprotein contained in milk, tears, saliva, blood, and so forth of mammals such as sheep, goat, pig, mouse, water buffalo, camel, yak, horse, donkey, llama, cow, and human.

Although lactoferrin used for the present invention may originate in any mammal, and is not particularly limited, for example, lactoferrin derived from the milk of a cow, human, or the like is preferred in view of content and availability. The milk may be any of colostrum, transitional milk, nature milk, and late lactation milk.

Lactoferrin used for the present invention may be lactoferrin separated from skim milk, whey, and so forth, which are processed products of the milk, in a conventional manner (for example, ion chromatography etc.), recombinant lactoferrin produced by a genetically manipulated microorganism, animal cell, transgenic animal, or the like, synthetic lactoferrin, or a mixture of these. Lactoferrin may be a deglycosylated or glycosylated lactoferrin. As such lactoferrin, marketed lactoferrin produced on an industrial scale (produced by, for example, Morinaga Milk Industry Co., Ltd.) can be used.

The metal content of lactoferrin used for the present invention is not particularly limited, and any one of apolactoferrin obtained by deferrization of lactoferrin with hydrochloric acid, citric acid, or the like; metal-saturated lactoferrin in a saturation degree of 100% or more obtained by chelating the apolactoferrin with a metal such as iron, copper, zinc, and manganese; and partially metal-saturated lactoferrin in which metal binds at a saturation degree lower than 100%, or a mixture consisting of two or more of these can be used.

Hereafter, an example of a method for preparing lactoferrin (separation and purification of lactoferrin from a raw material such as milk) used for the present invention will be explained below. However, the method is not limited to this method.

A milk raw material derived from cow is loaded on a cation exchange column, the eluate is collected, and this eluate is repeatedly loaded on the column as required. Deionized water is loaded on this column, and an aqueous sodium chloride solution is further loaded to obtain an eluate containing basic proteins having been adsorbed onto the cation exchange column. The proteins are collected from this eluate by ammonium sulfate precipitation and washed as required. The collected precipitates are dissolved in deionized water, and this solution is filtered through an ultrafiltration membrane. The residue can be further subjected to desalting and lyophilization to obtain powdered lactoferrin.

More precisely, the column is filled with an ion exchanger, hydrochloric acid is loaded, and the ion exchanger is washed with water and thereby equilibrated. Then, skim milk of pH 6.9 cooled to 4°C is loaded onto the column, and the eluate is collected, and similarly loaded on the column again. Subsequently, deionized water is loaded on the column, and an aqueous sodium chloride solution is further loaded to obtain an eluate containing basic proteins having been adsorbed on the cation exchange column. Ammonium sulfate at a saturation degree of 80% is added to this eluate to precipitate the proteins, and the precipitates are collected by centrifugation. The collected precipitates are washed with an 80% saturated ammonium sulfate solution, and deionized water is added to dissolve the precipitates; the obtained solution is desalted by using an ultrafiltration membrane module, and the residue is lyophilized to obtain powdered bovine lactoferrin. Bovine lactoferrin having a purity of 95 mass % or higher can be obtained as described above.

Lactoferrin genes naturally contain a genetic mutation such as a substitution, deletion, insertion and inversion of one or more nucleotides at one or more positions depending on the difference of species, genus, individual, etc., and amino acid sequence of a protein encoded by a gene containing such mutations may also contain mutations. Lactoferrin used for the present invention may be lactoferrin containing such a mutation, so long as the effect of the present invention is not degraded.

Lactoferrin used for the present invention may also be a processed product of lactoferrin obtained by heat treatment, acid treatment, or alkaline treatment of lactoferrin, so long as the effect of the present invention is not degraded.

The lactoferrin hydrolysate referred to in the present invention is such lactoferrin as mentioned above subjected to a hydrolysis treatment.

Examples of the hydrolysis treatment include, for example, the hydrolysis treatment by the method described in Japanese Patent Laid-open (KOKAI) No. 2012-235768.

Specifically, before a lactoferrin solution is subjected to an enzymatic reaction treatment, the solution is adjusted to pH 2 to 4, preferably pH 2.5 to 3.5, particularly preferably pH 3, with an acid such as hydrochloric acid, citric acid, and acetic acid.

An enzyme composition containing chymosin is added in a desired amount to the lactoferrin solution of which pH has been adjusted, and then the temperature is maintained for the enzymatic reaction at 35 to 55°C, preferably 40 to 50°C, more preferably 42 to 48°C, with stirring to hydrolyze lactoferrin for 6 to 24 hours, preferably 12 to 18 hours.

Subsequently, the temperature of the reaction solution is elevated to, for example, 80°C, and maintained at that temperature for 10 minutes to carry out heat inactivation of the enzyme. An alkali solution such as sodium hydroxide solution is preferably further added to adjust the solution to pH 5 to 7, for example, pH 6.

Although the reaction solution (lactoferrin hydrolysate) of which pH has been adjusted may be used as solution, it is preferably powdered by lyophilization, or the like. As the lactoferrin hydrolysate, a fractionation product of the reaction solution obtained by chromatography, ultrafiltration, or the like can also be used.

In the nutritional composition of the present invention, content of lactoferrin and/or lactoferrin hydrolysate based on the entire nutritional composition is preferably 0.001 to 5.0 mass %, more preferably 0.005 to 1.0 mass %, more preferably 0.01 to 0.5 mass %, particularly preferably 0.01 to 0.2 mass %.

These ranges may usually be the ranges of the content in the nutritional composition that is to be distributed as a commercial product.

The human milk oligosaccharide used for the present invention is not particularly limited so long as it is an oligosaccharide usually contained in human milk, but preferred examples include neutral human milk oligosaccharides such as 2'-fucosyllactose, 3-fucosyllactose, lactodifucotetraose, 2',3-difucosyllactose, lacto-N-triose II, lacto-N-tetraose, lacto-N-neotetraose, lacto-N-fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose, and lacto-N-neohexaose, and acidic human milk oligosaccharides such as 3'-sialyllactose, 6'-sialyllactose, 3-fucosyl-3'-sialyllactose, and disialyl-lacto-N-tetraose. Among these, 2'-fucosyllactose, 3-fucosyllactose, 3'-sialyllactose, and 6'-sialyllactose are preferred, and 2'-fucosyllactose is particularly preferred.

The human milk oligosaccharide used for the present invention may be a purified oligosaccharide or may be a mixture so long as the effect of the present invention is not degraded. And the human milk oligosaccharide used for the present invention may contain one or more of the human milk oligosaccharides mentioned above.

In the nutritional composition of the present invention, the content of the human milk oligosaccharide based on the entire nutritional composition is preferably 0.001 to 10.0 mass %, more preferably 0.005 to 5.0 mass %, further preferably 0.005 to 2.0 mass %, particularly preferably 0.01 to 2.0 mass %.

In the composition of the present invention, mass ratio of the human milk oligosaccharide to lactoferrin and/or lactoferrin hydrolysate is preferably not lower than 1/100 and not higher than 100, more preferably not lower than 1/10 and not higher than 100, further preferably not lower than 1/5 and not higher than 50.

These ranges may usually be the ranges of the content in the nutritional composition that is to be distributed as a commercial product.

The contents at the time of ingestion (administration) of the nutritional composition of the present invention may be within the aforementioned ranges, or the composition may be, for example, diluted, as required. For example, the content of lactoferrin and/or lactoferrin hydrolysate based on the entire composition at the time of ingestion (administration) can be 0.0001 to 10.0 mass %. Further, the content of the human milk oligosaccharide based on the entire composition at the time of ingestion (administration) can be 0.0001 to 10.0 mass %. The mass ratio of the human milk oligosaccharide to lactoferrin and/or lactoferrin hydrolysate at the time of ingestion (administration) can be not lower than 1/100 and not higher than 100.

The nutritional composition of the present invention may further contain a probiotic.

As the probiotic used for the present invention, *Lactobacillus* bacteria, *Streptococcus* bacteria, and *Bifidobacterium* bacteria, which are resident lactic acid bacteria in the human alimentary canal, can be mainly used. In particular, lactic acid bacteria of the genus *Lactobacillus* are most frequently used. Especially, *Lactobacillus rhamnosus* GG strain (Japanese Patent Laid-open (KOKAI) 61-280433), *Lactobacillus casei* Shirota strain (marketed by Yakult Honsha Co., Ltd.), *Lactobacillus johnsonii* La1 strain (Japanese Patent Laid-open (KOKAI) No. 6-315373), *Lactobacillus plantarum* 299 strain (DSM6595) (Japanese Patent Laid-open (KOHYO) No. 6-501624), *Lactobacillus plantarum* 299v strain (DSM9843) (Japanese Patent Laid-open (KOHYO) No. 11-502703), *Lactobacillus salivarius* UCC 1 strain (NCIMB 40830), *Lactobacillus salivarius* UCC 118 strain (NCIMB 40829, WO98/35014), and so forth can used as strains suitable for probiotic products.

The *Lactobacillus* bacteria are not particularly limited, and examples include *Lacticaseibacillus casei, Lacticaseibacillus paracasei, Lacticaseibacillus rhamnosus, Lactiplantibacillus plantarum, Levilactobacillus brevis, Ligilactobacillus salivarius, Limosilactobacillus fermentum, Limosilactobacillus reuteri.*

In the present invention, *Bifidobacterium* bacteria are still more preferably used as the probiotic.

The *Bifidobacterium* bacteria are not particularly limited, and examples include *Bifidobacterium breve, Bifidobacterium infantis* (later reclassified as *Bifidobacterium longum* subsp. *infantis*)*, Bifidobacterium longum, Bifidobacterium bifidum, Bifidobacterium adolescentis, Bifidobacterium catenulatum, Bifidobacterium pseudocatenulatum, Bifidobacterium animalis, Bifidobacterium lactis,* and *Bifidobacterium pseudolongum.* Among these, one or more of *Bifidobacterium breve, Bifidobacterium infantis,* and *Bifidobacterium longum* are preferred.

In the present invention, the probiotic, more preferably *Bifidobacterium* bacteria, may consist of live cells or dead cells. When live cells are used, 1 mL of the nutritional composition preferably contains 1.0 × 10⁶ cfu or more, more preferably 1.0 × 10⁷ cfu or more, further preferably 2.0 × 10⁷ cfu or more, of live cells. When live cells are used, dead cells may also be present.

The unit "cfu" means "colony-forming unit." In this description, the values of this unit may be, for example, those obtained in a culture at 38°C on a solid medium containing 10 mass % of reduced skim milk powder.

Preferred examples of the *Bifidobacterium* bacteria to be present in the nutritional composition of the present invention are one or more of *Bifidobacterium breve, Bifidobacterium infantis,* and *Bifidobacterium longum.*

More specifically, examples of *Bifidobacterium breve* include *Bifidobacterium breve* M-16V. *Bifidobacterium breve* M-16V was deposited on January 26, 2018 at the independent administrative agency, National Institute of Technology and Evaluation, NITE Patent Microorganisms Depositary (NPMD, #122, 2-5-8, Kazusa Kamatari, Kisarazu-shi, Chiba-ken, 292-0818, Japan) as an international deposit under the provisions of the Budapest Treaty, and given the accession number of NITE BP-02622. Commercially available products, for example, *"Bifidobacterium breve* M-16V" produced by Morinaga Milk Industry Co., Ltd., may be used.

More specifically, examples of *Bifidobacterium breve* also include *Bifidobacterium breve* MCC1274. *Bifidobacterium breve* MCC1274 was deposited on August 25, 2009 at the independent administrative agency, National Institute of Advanced Industrial Science and Technology, International Patent Organism Depositary (currently, the independent administrative agency, National Institute of Technology and Evaluation, International Patent Organism Depositary (IPOD, #120, 2-5-8, Kazusa Kamatari, Kisarazu-shi, Chiba-ken, 292-0818, Japan) as an international deposit under the provisions of the Budapest Treaty, and given the accession number of FERM BP-11175.

More specifically, examples of *Bifidobacterium infantis* also include *Bifidobacterium infantis* M63. *Bifidobacterium infantis* M63 was deposited on January 26, 2018 at NPMD as an international deposit under the provisions of the Budapest Treaty, and given the accession number of NITE BP-02623.

More specifically, as *Bifidobacterium longum, Bifidobacterium longum* NITE BP-02621 (synonym: BB536 or *Bifidobacterium longum* subsp. *longum* ATCC BAA-999) can be used. *Bifidobacterium longum* BB536 was deposited at NPMD on January 26, 2018 as an international deposit under the provisions of the Budapest Treaty, and given the accession number of NITE BP-02621. The same bacterial strain, *Bifidobacterium longum* subsp. *longum* ATCC BAA-999 (ATCC BAA-999), can be obtained from American Type Culture Collection (ATCC, 10801 University Boulevard, Manassas, VA 20110, United States of America) as ATCC BAA-999 (see, for example, Japanese Patent Laid-open (KOKAI) No. 2012-223134 etc.).

The bacteria identified by the names of the bacteria exemplified above are not limited to the strains deposited or registered at the depositaries mentioned above themselves (henceforth referred to as "deposited strains" for convenience of explanation), and may be strains substantially equivalent to the deposited strains (also referred to as "derived strains" or "derivative strains"). The "strains substantially equivalent to the aforementioned deposited strains" refer to strains that belong to the same species as those of the aforementioned deposited strains, provide an intestinal bacterial flora-improving effect, preferably have a nucleotide sequence of the 16SrRNA gene showing an identity of 99.86% or higher, more preferably 99.93% or higher, further preferably 100%, to the nucleotide sequence of 16SrRNA genes of the aforementioned deposited strains, and preferably have the same bacteriological characteristics as those of the aforementioned deposited strains, respectively. The strains substantially equivalent to the aforementioned deposited strains may be, for example, derivative strains derived from the respective deposited strains as a parent strain. Examples of the derivative strains include a strain bred from any of the deposited strains, and a strain naturally produced from any of the deposited strains. Examples of the breeding method include modification based on a genetic engineering technique and modification caused by using a mutation treatment. Examples of the mutation treatment include irradiation of X-rays, irradiation of ultraviolet rays, and treatment with a mutation agent such as N-methyl-N'-nitro-N-nitrosoguanidine, ethyl methanesulfonate, and methyl methanesulfonate. Examples of the strain naturally produced from any of the deposited strains include a strain naturally produced at the time of use of any of the deposited strains. Examples of such a strain include a mutant strain naturally produced in culture (for example, subculture) of any of the deposited strains. The derivative strains may be constructed by one type of modification, or may be constructed by two more types of modifications.

As cells of *Bifidobacterium* bacteria that may be present in the nutritional composition of the present invention, commercial products may be used, or those obtained by an appropriate production method may be used.

Cells of *Bifidobacterium* bacteria that may be present in the nutritional composition of the present invention can also be easily obtained by culturing any of the aforementioned *Bifidobacterium* bacteria. The culture method is not particularly limited so long as *Bifidobacterium* bacteria can be proliferated. As the culture method, for example, methods usually used for culturing *Bifidobacterium* bacteria can be used as they are, or with appropriate modifications. The culture temperature may be, for example, 25 to 50°C, preferably 35 to 42°C. The culture can be preferably carried out under anaerobic conditions, and for example, it can be carried out by supplying an anaerobic gas such as carbon dioxide gas. The culture can also be carried out under microaerobic conditions such as those obtainable by stationary liquid culture or the like. The culture can be carried out, for example, until *Bifidobacterium* bacteria proliferate to a desired level.

The medium used for the culture is not particularly limited so long as *Bifidobacterium* bacteria can be proliferated. As the medium, for example, media usually used for culturing *Bifidobacterium* bacteria can be used as they are or with appropriate modifications. That is, as the carbon source, for example, saccharides such as galactose, glucose, fructose, mannose, cellobiose, maltose, lactose, sucrose, trehalose, starch, starch hydrolysate, and blackstrap molasses can be used depending on assimilation property of the bacteria. As the nitrogen source, for example, ammonia, ammonium salts such as ammonium sulfate, ammonium chloride, and ammonium nitrate, and nitrates can be used. As inorganic salts, for example, sodium chloride, potassium chloride, potassium phosphate, magnesium sulfate, calcium chloride, calcium nitrate, manganese chloride, ferrous sulfate, and so forth can be used. Organic components such as peptone, soybean flour, defatted soybean meal, meat extract, and yeast extract may also be used. Specifically, examples of media usually used for culturing *Bifidobacterium* bacteria include the reinforced clostridial medium, MRS medium (de Man, Rogosa, and Sharpe medium), mMRS medium (modified MRS medium), TOSP medium (TOS propionate medium), and TOSP Mup medium (TOS propionate mupirocin medium).

As *Bifidobacterium* bacteria that may be contained in the nutritional composition of the present invention, cells of the bacteria or a fraction containing the cells can be used without any particular limitation. That is, as *Bifidobacterium* bacteria, for example, a culture obtained by culturing the bacteria can be used as it is, such culture may be used after dilution or concentration, or cells collected from such culture may be used. So long as the intestinal bacterial flora-improving effect is not degraded, the culture product may be subjected to any of various additional treatments such as heating and lyophilization after the culture. Additional treatments that allow higher survivability of the cells are more preferred. That is, specific examples of *Bifidobacterium* bacteria that may be present in the nutritional composition of the present invention include culture of *Bifidobacterium* bacteria, cells collected from culture of *Bifidobacterium* bacteria, and processed products of these, and examples of the processed products include products obtained by dilution, concentration, drying, or the like. The cells usually preferably include live cells. The cells may consist of, for example, live cells or a mixture of live cells and dead cells.

The nutritional composition of the present invention may further contain a prebiotic.

The term "prebiotic" may indicate an indigestible food component that allows selective proliferation of useful bacteria in the intestinal tract. Examples of such useful bacteria include *Bifidobacterium* bacteria. That is, as the prebiotic, an ingredient that selectively proliferates *Bifidobacterium* bacteria is preferred. As the prebiotic, one kind of ingredient may be used, or a combination of two or more kinds of ingredients may be used.

The prebiotic is not particularly limited by, specifically, a probiotic concomitantly used, so long as the desired effect can be obtained. Examples of the prebiotic include oligosaccharides, dietary fibers, and gluconic acid. Examples of the oligosaccharides include galactooligosaccharides, fructooligosaccharides, xylooligosaccharide, isomaltooligosaccharides, raffinose, lactulose, lactosucrose, soybean oligosaccharides, and coffee oligosaccharides.

The nutritional composition of the present invention can be used for treating an infectious disease or pathological condition, wherein the nutritional composition can prevent infection or stimulate immunity. It can be used especially as a nutritional composition for preventing viral infection.

The expression "for prevention of viral infection" used for the present invention usually indicates a use for making infants, young children and elderly persons with low resistance who are easily infected with viruses, exhibit an effect of preventing infection with virus, or protecting them from the infection. Examples of viruses for which such a protection effect for the infection thereof can be exhibited include rotaviruses and most viruses of the family *Reoviridae* to which rotaviruses belong, influenza viruses, noroviruses, cytomegaloviruses, RS viruses, polioviruses, and HIV.

The expression "action for preventing infection" used in this description refers to an action of the nutritional composition of the present invention of preventing viral infection or the action of mitigating the pathological symptoms in infected patients, through ingestion of the composition before and during the epidemic period of viral infection.

The nutritional composition of the present invention can be preferably used for the prevention of viral infection in the gastrointestinal tract. Specific examples of virus that can infect gastrointestinal tract include rotaviruses and noroviruses. It is considered that the action mechanism of the prevention of infection by the nutritional composition of the present invention is based on suppression of adhesion of the viruses with cells, specifically, cells of gastrointestinal tract, more specifically, intestinal epithelial cells. It is also considered that the action mechanism of the prevention of infection (including reduction of severity) by the nutritional composition of the present invention is also based on suppression of gene replication of viruses in vivo after the viral infection.

By adding an effective amount of the nutritional composition as a nutritional composition for childcare of infants, young children with immature defensive ability, or a food or nutritional composition for elderly persons with reduced defensive ability against infection, a nutritional composition for preventing infection can be prepared.

Another aspect of the present invention is the use of lactoferrin and/or a lactoferrin hydrolysate, and a human milk oligosaccharide in the manufacture of a nutritional composition for preventing infection.

Still another aspect of the present invention is use of a nutritional composition containing lactoferrin and/or a lactoferrin hydrolysate, and a human milk oligosaccharide for preventing infection.

Still another aspect of the present invention is a nutritional composition containing lactoferrin and/or a lactoferrin hydrolysate, and a human milk oligosaccharide, which is used for preventing infection.

Still another aspect of the present invention is a method for preventing infection, which includes administering lactoferrin and/or a lactoferrin hydrolysate, and a human milk oligosaccharide to an animal or human. Although the object of the administration is not particularly limited, it is usually a human.

The ingestion (administration) period of the nutritional composition of the present invention is not particularly limited and can be appropriately chosen according to the condition of the object of the administration.

The ingestion (administration) amount of the nutritional composition of the present invention is appropriately chosen according to age, sex, pathological condition, other conditions, and so forth of a subject of the ingestion (object of administration).

The nutritional composition of the present invention is preferably prepared in the form of food or drink.

Forms and properties of the food and drink are not particularly limited so long as the effect of the present invention is not degraded, and they can be prepared by usual methods using raw materials usually used for foods and drinks.

The nutritional composition in the form of an additive to be added to a food or drink also falls within the scope of the composition of the present invention. Examples of this embodiment include, for example, additives to be added to collected mother's milk or formula milk, and ingestion of the milk containing the additive by neonates or infants is supposed.

Although food or drink is usually orally ingested, the ingestion route is not limited to this route, and for example, it may be ingested via a paranasal route, or ingested by using a gastric fistula or cecal fistula. For example, it is supposed that formula milk for neonates or infants, which is the composition of the present invention mentioned later, or mother's milk to which the composition of the present invention is added is ingested by neonates or infants through a paranasal stomach feeding tube, or the like.

Forms of the food and drink are not particularly limited, and they may be in the form of liquid, paste, gellated solid, powder, or the like. Examples include, for example, tablet confectioneries, and liquid diets (nutrition diets for tube feeding), as well as flour products such as bread, macaroni, spaghetti, noodles, cake mix, fry powder and bread crumbs; ready-to-eat foods such as instant noodles, cup noodles, retort and cooked foods, canned cooking, foods for microwave heating, instant soup and stew, instant miso soup and Japanese clear soup, canned soup, freeze-dried foods, and other ready-to-eat foods; processed agricultural products such as canned agricultural products, canned fruits, jams and marmalades, pickles, cooked beans, dry agricultural products, and cereals (processed grain products); processed marine products such as canned marine products, fish hams and sausages, seafood paste products, marine dainties, and tsukudani (marine products boiled in soy sauce); processed livestock products such as canned livestock products and pastes, and livestock meat hams and sausages; milks and dairy products such as processed milk, milk drinks, yoghurts, lactic acid drinks, cheese, ice creams, creams, and other dairy products; oils and fats such as butters, margarines, and vegetable oils; basic seasonings such as soy sauce, miso, sauces, processed tomato seasoning, mirin, and vinegars; complex seasonings and foods such as cooking mix, curry powder or roux, sauces for dipping, dressings, noodle soups, spices, and other complex seasonings; frozen foods such as frozen food materials, semi-cooked frozen foods, and cooked frozen foods; confectioneries such as caramels, candies, chewing gums, chocolates, cookies, biscuits, cakes, pies, snacks, crackers, Japanese sweets, rice confectioneries, bean confectioneries, dessert pastries, jellies, and other confectioneries; beverages such as carbonated drinks, natural fruit juices, fruit juice drinks, fruit juice soft drinks, fruit pulp drinks, fruit drinks with fruit pulp, vegetable based drinks, soy milk, soy milk drinks, coffee drinks, tea drinks, powdered drinks, concentrated drinks, sports drinks, energy drinks, alcoholic drinks, and other beverages; other commercial foods such as baby foods, rice seasonings, and seaweed seasonings for boiled rice soaked with tea; nutritional compositions such as supplements and formula milk (including powdered formula and liquid formula); nutritional compositions for infants such as supplements for infants and infant formula(including powdered formula and liquid formula); enteral nutrition; functional foods (foods for specified health uses, foods with nutrient function claims), and so forth.

Among these, a nutritional composition for infants and children is preferred.

In Japan, the term nutritional composition for infants and children refers to a nutritional composition for 0 to 12 months old infants (also referred to as infant formula), follow-up milk for 6 to 9 months old infants and children (up to 6 years old) (also referred to as formula for infants and children), nutritional composition for low birth weight infants of birth weight lower than 2500 g (low birth weight infants) (also referred to as formula for premature or low birth weight infants), formulas for special medical purpose used for treating infants with pathological conditions such as cow's milk allergy and lactose intolerance, and so forth. The nutritional composition can also be applied to foods with health claims or foods for medical purpose.

Outside Japan, the term nutritional composition for infants and children refers to a nutritional composition for 0 to 6 month old infants (also referred to as infant formula), nutritional composition for 6 to 12 month old infants and for 1 to 3 year old children (also referred to as follow-up formula), nutritional composition for 3 to 6 year old children (also referred to as growing-up milk), nutritional composition for low birth weight infants of birth weight lower than 2500 g (also referred to as formula for premature or low birth weight infants), formulas for special medical purpose used for treating infants with pathological conditions such as cow's milk allergy and lactose intolerance, and so forth. The nutritional composition can also be applied to foods with health claims or foods for medical purpose.

Examples of the nutritional composition of the present invention also include balanced nutritional foods such as formula milk for pregnant and lactating mothers (formula milk containing nutrients required for gestation period and lactation period in good balance), formula milk for school-age children, formula milk for adults, and liquid foods for elderly persons, nutritional supplements, and foods for medical purpose such as phosphorus-reduced powdered milk (food for special medical purpose).

As an embodiment of food or drink, the nutritional composition of the present invention may be prepared as a feed. Examples of the feed include pet foods, feeds for livestock, feeds for fish breeding, and so forth.

Forms of the feeds are not particularly limited, and the feeds may be, for example, those containing cereals such as corn, wheat, barley, rye, and milo; vegetable oil cakes such as soybean oilcake, rapeseed oilcake, coconut oilcake, and linseed oilcake; brans such as wheat bran, rice bran, and defatted rice bran; manufacturing meals such as corn gluten meal and corn germ meal; animal feeds such as fish meal, skim milk powder, whey, yellow grease, and tallow; yeasts such as torula yeast and brewer's yeast; mineral feeds such as tribasic calcium phosphate and calcium carbonate; oils and fats; simple amino acids; saccharides, and so forth.

When the nutritional composition of the present invention is in the form of food or drink, it may be a food with health claims or a food for special dietary uses. The food with health claims system was provided not only for usual foods, but also for foods in the form of tablet, capsule, or the like in consideration of the domestic and foreign trends and the compatibility with the conventional food for specified health uses system, and foods with health claims are classified into three types, foods for specified health uses, foods with function claims, and foods with nutrient function claims. The foods for special dietary uses are foods for special uses for those who cannot eat usual meals such as sick persons, infants, young children, and elderly persons, and include foods for special medical purpose (standard regulation type and individual evaluation type), powdered formula for pregnant women and lactating mothers, infant formula, and foods for dysphagia.

When the composition of the present invention is in the form of food or drink (including feed), it can be provided and sold as a food or drink with an indication (labeling) of use for preventing infection.

The aforementioned term "indication" means all actions for informing consumers the aforementioned use, and any indications reminding or analogizing the aforementioned use fall within the scope of the "indication" referred to in the present invention regardless of purpose and content of the indication, objective article or medium on which the indication is used, and so forth.

The term "indication" is preferably made with an expression that allows consumers to directly recognize the aforementioned use. Specific examples include actions of assigning or delivering commodities including the food or drink on which the aforementioned use is indicated or such commodities having packages on which the aforementioned use is indicated, as well as displaying or importing such commodities, displaying or distributing advertisements, price lists or business papers relating to such commodities on which the aforementioned use is indicated, providing information including those as contents that indicate the aforementioned use by an electromagnetic method (Internet etc.) for the purpose of assigning or delivering such commodities, and so forth.

The indication is preferably an indication approved by an administration etc. (for example, an indication based on an approval, which is qualified on the basis of any of various legal systems provided by the government of Japan). Indications of such contents on packages, containers, catalogues, pamphlets, advertisement materials used at the point of sale, such as POPs, and other papers are preferred.

Examples of the "indication" include, for example, indications as health food, functional food, enteral nutritive food, food for special dietary uses, food with health claims, food for specified health uses, food with nutrient function claims, food with function claims, quasi-drug, and so forth. Among these, indications approved by the Consumer Affairs Agency, for example, indications approved on the basis of the systems of food for specified health uses, food with nutrient function claims, food with function claims, and similar systems can be especially mentioned. Specific examples include indications as food for specified health uses, indications as food for specified health uses with qualified health claims, indications of influence on body structures and functions, indications of reduction of disease risk claims, indications of functional claims based on scientific evidences, and so forth, and more precisely, typical examples include the indications as food for specified health uses (especially indications of use for health) defined in the Cabinet Office Ordinance concerning approval of indications of special dietary uses provided by the Health Promotion Law, and others (Japanese Cabinet Office Ordinance No. 57, August 31, 2009), and similar indications.

As such an indication, for example, wordings of "for preventing infection", "measure against viral infection", "for infants' health", and so forth can be indicated.

The composition of the present invention can also be in the form of drug, and in this case, as for the administration route thereof, it may be orally or parenterally administered, but it is preferably orally administered. Examples of parenteral ingestion (administration) include rectal administration, and so forth.

As for form of the drug, it can be appropriately prepared in a desired dosage form depending on the administration method. For example, in the case of oral administration, it can be prepared in the form of solid preparation such as a powder, granule, tablet, and capsule; liquid agent such as a solution, syrup, suspension, and emulsion, or the like. In the case of parenteral administration, it can be prepared in the form of a suppository, ointment, injection, or the like.

When the drug is prepared, such ingredients as excipients, pH adjustors, colorants and corrigents, which are usually used for the preparation of drugs, can be used. Another pharmaceutical ingredient, a prebiotic for *Bifidobacterium* bacteria that is known, or one that is discovered in the future, or the like may also be used together.

The drug can be prepared by a known method appropriately chosen depending on the dosage form. The drug may also be prepared by adding a carrier for pharmaceutical compositions.

Examples of excipients include, for example, saccharides and derivatives thereof such as lactose, sucrose, glucose, mannitol, and sorbitol; starch and derivatives thereof such as corn starch, potato starch, pregelatinized starch, dextrin, and carboxymethyl starch; cellulose and derivatives thereof such as crystalline cellulose, hydroxypropylcellulose, hydroxypropylmethylcellulose, carboxymethylcellulose and carboxymethylcellulose calcium; gum arabic; dextran; pullulan; silicate and derivatives thereof such as light silicic anhydride, synthetic aluminum silicate and magnesium metasilicate aluminate; phosphate and derivatives thereof such as calcium phosphate; carbonate and derivatives thereof such as calcium carbonate; sulfate and derivatives thereof such as calcium sulfate, and so forth.

Examples of binders include, in addition to the aforementioned excipients, for example, gelatin; polyvinylpyrrolidone; Macrogol, and so forth.

Examples of disintegrating agents include, in addition to the aforementioned excipients, for example, chemically modified starch or cellulose derivatives such as croscarmellose sodium, and carboxymethylstarch sodium, crosslinked polyvinylpyrrolidone, and so forth.

Examples of lubricants include, for example, talc; stearic acid; stearic acid metal salts such as calcium stearate and magnesium stearate; colloidal silica; waxes such as peegum and spermaceti; boric acid; glycols; carboxylic acids such as fumaric acid and adipic acid; carboxylic acid sodium salts such as sodium benzoate; sulfates such as sodium sulfate; leucine; laurylsulfates such as sodium laurylsulfate and magnesium laurylsulfate; silicic acids such as silicic anhydride and silicic acid hydrate; starch derivatives, and so forth.

Examples of stabilizers include, for example, p-oxybenzoic acid esters such as methylparaben and propylparaben; alcohols such as chlorobutanol, benzyl alcohol and phenylethyl alcohol; benzalkonium chloride; acetic anhydride; sorbic acid, and so forth.

Examples of corrigents include, for example, sweeteners, acidulants, perfumes, and so forth.

Examples of carriers used for liquids for oral administration include solvents such as water, and so forth.

### Examples

Hereafter, the present invention will be explained more in detail with reference to examples. However, the present invention is not limited by these examples.

### [Example 1] Examination of antiviral action exhibited by lactoferrin, human milk oligosaccharide, or both against simian rotavirus

The following experiment was performed to evaluate the antiviral action exhibited by lactoferrin, human milk oligosaccharide, or both against rotavirus.

The simian rotavirus SA11 strain (Nihon Bioresearch Inc.) was inoculated to the MA104 cells (Sigma-Aldrich) of African green monkey kidney maintained in a minimum essential medium (Sigma-Aldrich, henceforth referred to simply as "medium"). The simian rotavirus SA11 strain, for example, can be obtained from American Type Culture Collection (ATCC) as ATCC VR-1565. To the medium, lactoferrin (LF) and/or the human milk oligosaccharide(s) shown below were added beforehand alone or in combination at the following concentrations, respectively (n = 3).
LF (produced by Morinaga Milk Industry Co., Ltd.): 0, 0.1 or 1.0 mg/mL
2'-Fucosyllactose (2'-FL, Funakoshi Co., Ltd.): 0, 0.1, 0.5, or 2.5 mg/mL
3-Fucosyllactose (3-FL, Funakoshi Co., Ltd.): 0, 0.1, or 0.5 mg/mL
3'-Sialyllactose (3'-SL, Funakoshi Co., Ltd.): 0, 0.1, or 0.5 mg/mL
6'-Sialyllactose (6'-SL, Funakoshi Co., Ltd.): 0, 0.1, or 0.5 mg/mL
Human milk oligosaccharides (mixture) (HMO mix, mixture of 2'-FL (2.5 mg/mL), 3-FL (0.5 mg/mL), 3'-SL (0.25 mg/mL) and 6'-SL (0.5 mg/mL))

The amount of 1.0 mg/mL corresponds to 0.1 mass % in mass percentage.

Degree of infection was evaluated based on the number of "plaques" formed when the cells were infected with the virus. Specifically, the antiviral effects of lactoferrin and human milk oligosaccharides were evaluated based on "plaque-suppressing ratio (%)", i.e., the ratio of "plaque counts observed with lactoferrin and/or human milk oligosaccharide(s) supplementation" to "plaque counts observed without these supplementation". Plaque-suppressing ratio was then regarded as "infection-suppressing ratio (%)".

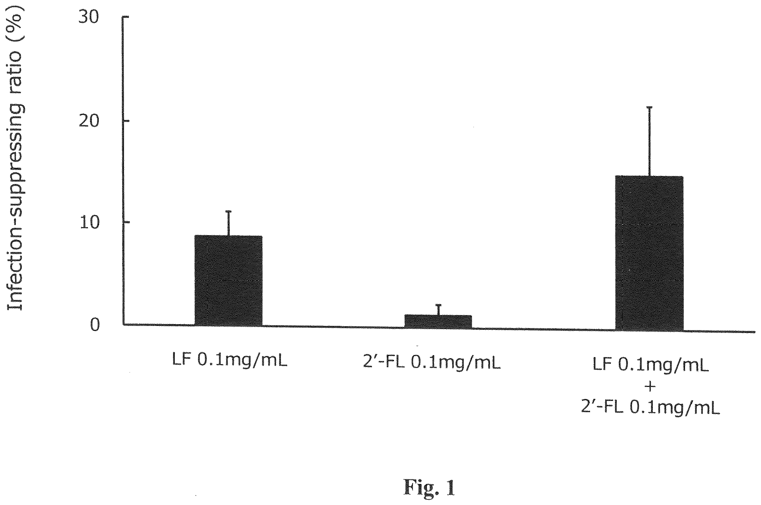

The results are shown in Figs. 1 to 7.

As shown in Fig. 1, the infection-suppressing ratio was 8.7% when LF was used alone in an amount of 0.1 mg/mL, or 1.2% when 2'-FL was used alone in an amount of 0.1 mg/mL, but it was 15% when 0.1 mg/mL of LF and 0.1 mg/mL of 2'-FL were used in combination.

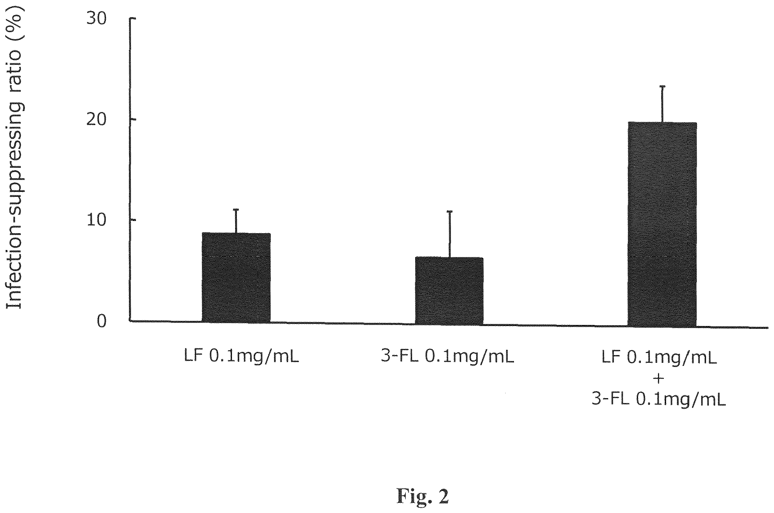

As shown in Fig. 2, the infection-suppressing ratio was 8.7% when LF was used alone in an amount of 0.1 mg/mL, or 6.6% when 3-FL was used alone in an amount of 0.1 mg/mL, but it was 20% when 0.1 mg/mL of LF and 0.1 mg/mL of 3-FL were used in combination.

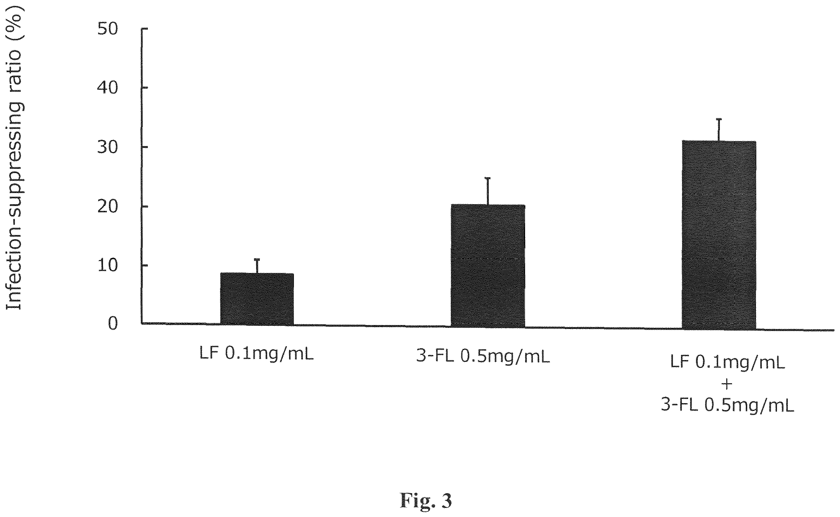

As shown in Fig. 3, the infection-suppressing ratio was 8.7% when LF was used alone in an amount of 0.1 mg/mL, or 21% when 3-FL was used alone in an amount of 0.5 mg/mL, but it was 32% when 0.1 mg/mL of LF and 0.5 mg/mL of 3-FL were used in combination.

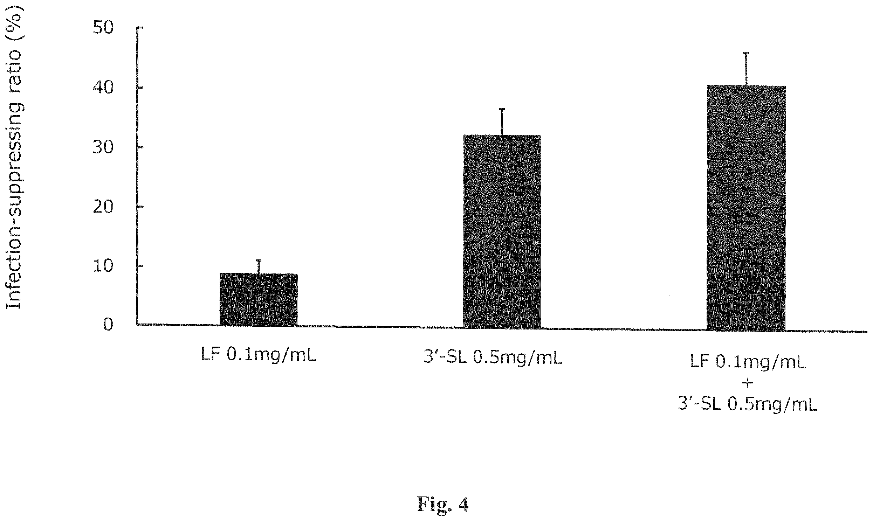

As shown in Fig. 4, the infection-suppressing ratio was 8.7% when LF was used alone in an amount of 0.1 mg/mL, or 33% when 3'-SL was used alone in an amount of 0.5 mg/mL, but it was 41% when 0.1 mg/mL of LF and 0.5 mg/mL of 3'-SL were used in combination.

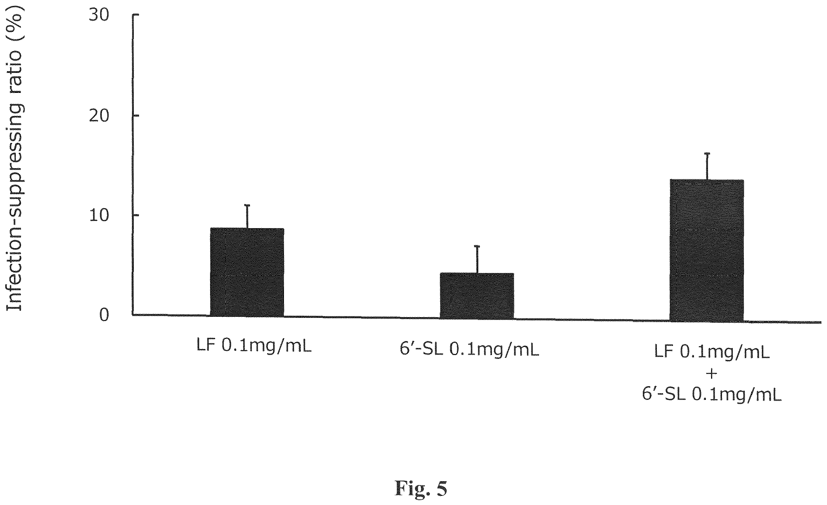

As shown in Fig. 5, the infection-suppressing ratio was 8.7% when LF was used alone in an amount of 0.1 mg/mL, or 4.5% when 6'-SL was used alone in an amount of 0.1 mg/mL, but it was 14% when 0.1 mg/mL of LF and 0.1 mg/mL of 6'-SL were used in combination.

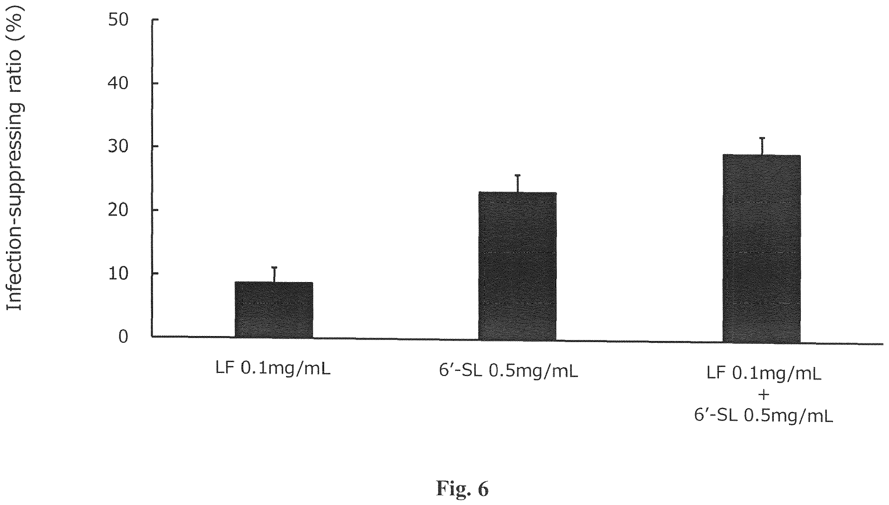

As shown in Fig. 6, the infection-suppressing ratio was 8.7% when LF was used alone in an amount of 0.1 mg/mL, or 23% when 6'-SL was used alone in an amount of 0.5 mg/mL, but it was 30% when 0.1 mg/mL of LF and 0.5 mg/mL of 6'-SL were used in combination.

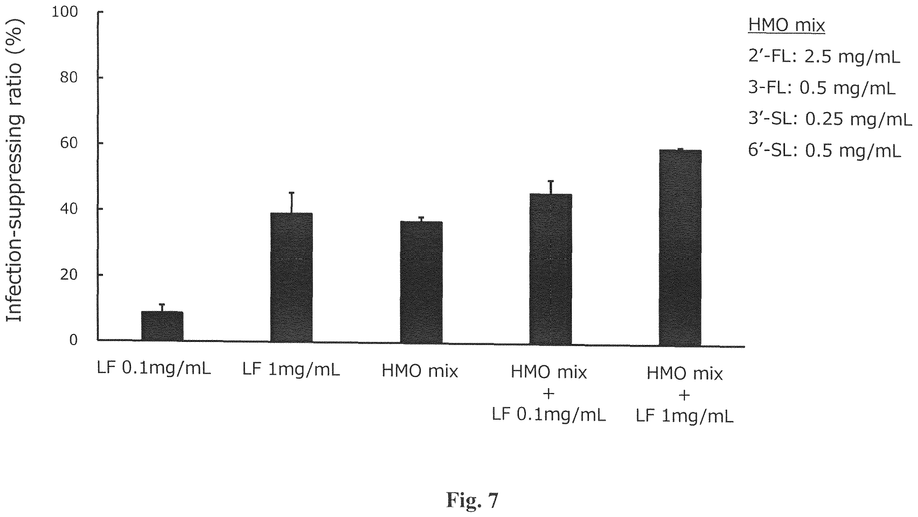

As shown in Fig. 7, the infection-suppressing ratio was 8.7% when LF was used alone in an amount of 0.1 mg/mL, or 37% when the HMO mix was used alone, but it was 46% when 0.1 mg/mL of LF and the HMO mix were used in combination. Further, the infection-suppressing ratio was 39% when LF was used alone in an amount of 1.0 mg/mL, but it was 60% when 1.0 mg/mL of LF and the HMO mix were used in combination.

As described above, when lactoferrin and human milk oligosaccharides were added in combination, higher plaque-suppressing ratios (infection-suppressing ratio) were observed compared with those observed with adding lactoferrin alone or human milk oligosaccharides alone. Therefore, it was recognized that the combination of lactoferrin and human milk oligosaccharides can exhibit synergistic or additive antiviral action against rotavirus.

### [Preparation Example 1]

A solution obtained by simultaneously dissolving lactoferrin (produced by Morinaga Milk Industry Co., Ltd.) and human milk oligosaccharides in water is lyophilized to obtain lyophilized powder of a mixture of lactoferrin and human milk oligosaccharides. The powder and whey protein concentrate (WPC, produced by Morinaga Milk Industry Co., Ltd.) are uniformly mixed to obtain a composition. This composition (20 g) is dissolved in water (200 g) to obtain a nutritional composition for preventing viral infection.

By administering the nutritional composition produced as described above to a target person, viral infection of the target person can be prevented.

### [Preparation Example 2]

A solution obtained by simultaneously dissolving lactoferrin (produced by Morinaga Milk Industry Co., Ltd.) and human milk oligosaccharides in water is lyophilized to obtain a lyophilized powder of a mixture of lactoferrin and human milk oligosaccharides. The powder and dry powder of milk protein concentrate (MPC480, produced by Fonterra Limited, protein content 80%, casein : whey proteins = about 8:2)) are uniformly mixed to obtain a composition. This composition (20 g) is dissolved in water (200 g) to obtain a nutritional composition for preventing viral infection.

By administering the nutritional composition produced as described above to a target person, viral infection of the target person can be prevented.

### [Preparation Example 3]

A solution obtained by simultaneously dissolving lactoferrin (produced by Morinaga Milk Industry Co., Ltd.) and human milk oligosaccharides in water is lyophilized to obtain lyophilized powder of a mixture of lactoferrin and human milk oligosaccharides. Subsequently, the powder and crystalline cellulose are put into a stirring granulation machine, and mixed. Then, purified water is added, the mixture is granulated, and the granulation product is dried to obtain a granulated nutritional composition containing lactoferrin and human milk oligosaccharides, as well as the excipient.

By administering the nutritional composition produced as described above to a target person, viral infection of the target person can be prevented.

### [Preparation Example 4]

A method for producing fermented milk to which lactoferrin and human milk oligosaccharides are added will be explained below.

First, a milk raw material, lactoferrin (produced by Morinaga Milk Industry Co., Ltd.), and human milk oligosaccharides, as well as water, other ingredients, etc. as required are mixed, preferably homogenized, and sterilized by heating. The homogenization and heat sterilization treatments can be performed by conventional methods. A lactic starter is added (inoculated) to the heat-pasteurized sterilized modified milk liquid, and the modified milk liquid is maintained at a predetermined fermentation temperature and thereby fermented to obtain a fermentation product. Curd is formed by the fermentation.

As the lactic starter, for example, lactic acid bacteria usually used for yogurt production such as *Lactobacillus bulgaricus, Lactococcus lactis,* and *Streptococcus thermophilus* can be used. If pH reaches a target value, the formed curd is crushed by stirring, and cooled to 10°C or lower to obtain fermented milk. By cooling to 10°C or lower, the activity of the lactic acid bacterium can be reduced, and generation of the acid can be suppressed.

Concentrated fermented milk can further be obtained by the following method. The concentration step can be performed by appropriately using a known concentration method. For example, a centrifugation method or membrane separation method can be used. By the centrifugation method, whey in the object of the concentration (fermentation product containing lactoferrin and human milk oligosaccharides) is removed, and concentrated fermented milk containing lactoferrin and human milk oligosaccharides can be obtained with a higher solid concentration.

A fermented milk containing lactoferrin and human milk oligosaccharides according to the present invention can be produced as described above.

By administering the fermented milk produced as described above to a target person, viral infection of the target person can be prevented.

### [Preparation Example 5]

A method for producing a nutritional composition to which lactoferrin and human milk oligosaccharides are added will be explained below.

Desalted cow's milk whey protein powder (produced by Milei GmbH, 10 kg), milk casein powder (produced by Fonterra Limited, 6 kg), lactose (produced by Milei GmbH, 48 kg), mineral mixture (produced by Tomita Pharmaceutical Co., Ltd., 920 g), vitamin mixture (produced by Tanabe Seiyaku Co., Ltd., 32 g), lactoferrin (produced by Morinaga Milk Industry Co., Ltd., 17 to 690 g), and human milk oligosaccharides (68 to 1698 g) are dissolved in warm water (300 kg), the solution is heated at 90°C for 10 minutes to further dissolve the ingredients, modified fat (produced by TAIYO YUSHI CORP., 28 kg) is added to the solution, and the mixture is homogenized. Then, the homogenized mixture is subjected to sterilization and concentration steps, and spray-dried to prepare a nutritional composition containing lactoferrin and human milk oligosaccharides (about 95 kg). If the obtained nutritional composition is dissolved in water to prepare liquid formula having a total solid concentration of 14% (w/v), which is the standard solid concentration of formula milk, the lactoferrin concentration and human milk oligosaccharide concentration in the formula milk liquid will be 0.025 to 1 mg/mL and 0.1 to 5 mg/mL, respectively. By administering the nutritional composition produced as described above to an infant or young child, viral infection of the infant or young children can be prevented.

### [Preparation Example 6]

Lactoferrin (produced by Morinaga Milk Industry Co., Ltd.) is dissolved in purified water at a concentration of 5 mass %, and pH of the solution is adjusted to 3 with a hydrochloric acid solution to prepare a lactoferrin solution. The prepared lactoferrin solution is warmed to 45°C, then calf rennet (produced by RENCO Inc., containing 92 mass % of chymosin and 8 mass % of pepsin) is added in an amount of 3% based on the mass of lactoferrin, and the reaction is allowed to occur for 24 hours with stirring to cause hydrolysis.

After completion of the hydrolysis, the reaction mixture is warmed to 80°C, and maintained at the same temperature for 10 minutes to inactivate the enzymes.

The reaction mixture is cooled on ice, then a sodium hydroxide solution is added to the reaction mixture to adjust pH of the mixture to 6, and then the reaction mixture is lyophilized to produce lactoferrin hydrolysate powder.

A solution obtained by simultaneously dissolving the obtained lactoferrin hydrolysate powder and human milk oligosaccharides in water is lyophilized to obtain lyophilized powder of a mixture of the lactoferrin hydrolysate and human milk oligosaccharides. The powder and whey protein concentrate (WPC, produced by Morinaga Milk Industry Co., Ltd.) are uniformly mixed to obtain a composition. This composition (20 g) is dissolved in water (200 g) to obtain a nutritional composition for preventing viral infection.

By administering the nutritional composition produced as described above to a target person, viral infection of the target person can be prevented.

### [Preparation Example 7]

A method similar to the method for producing lactoferrin hydrolysate powder described in Preparation Example 6 is performed to produce lactoferrin hydrolysate powder.

Desalted cow's milk whey protein powder (produced by Milei GmbH, 10 kg), milk casein powder (produced by Fonterra Limited, 6 kg), lactose (produced by Milei GmbH, 48 kg), mineral mixture (produced by Tomita Pharmaceutical Co., Ltd., 920 g), vitamin mixture (produced by Tanabe Seiyaku Co., Ltd., 32 g), the obtained lactoferrin hydrolysate powder (17 to 690 g), and human milk oligosaccharides (68 to 1698 g) are dissolved in warm water (300 kg), the solution is heated at 90°C for 10 minutes to further dissolve the ingredients; modified fat (produced by TAIYO YUSHI CORP., 28 kg) is added to the solution, and the mixture is homogenized. Then, the homogenized mixture is subjected to sterilization and concentration steps, and spray-dried to prepare a nutritional composition containing lactoferrin hydrolysate and human milk oligosaccharides (about 95 kg). If the obtained nutritional composition is dissolved in water to prepare liquid formula having a total solid concentration of 14% (w/v), which is the standard solid concentration of formula milk, the lactoferrin hydrolysate concentration and human milk oligosaccharide concentration in the formula milk liquid will be 0.025 to 1 mg/mL and 0.1 to 5 mg/mL, respectively.

By administering the nutritional composition produced as described above to an infant or young child, viral infection of the infant or young children can be prevented.

## Claims

1. A nutritional composition containing lactoferrin and/or a lactoferrin hydrolysate, and a human milk oligosaccharide.

2. The nutritional composition according to claim 1, wherein mass ratio of the human milk oligosaccharide to the lactoferrin and/or lactoferrin hydrolysate is not lower than 1/100 and not higher than 100.

3. The nutritional composition according to claim 1 or 2, wherein content of the lactoferrin and/or lactoferrin hydrolysate is 0.001 to 5.0 mass % based on the whole composition.

4. The nutritional composition according to any one of claims 1 to 3, wherein content of the human milk oligosaccharide is 0.001 to 10.0 mass % based on the whole nutritional composition.

5. The nutritional composition according to any one of claims 1 to 4, wherein the human milk oligosaccharide contains one or more kinds selected from the group consisting of 2'-fucosyllactose, 3-fucosyllactose, 3'-sialyllactose, and 6'-sialyllactose.

6. The nutritional composition according to any one of claims 1 to 5, which is used for prevention of infection.

7. The nutritional composition according to claim 6, which is used for prevention of gastrointestinal tract infection.

8. The nutritional composition according to any one of claims 1 to 7, which is in the form of powdered formula.

9. The nutritional composition according to any one of claims 1 to 7, which is in the form of liquid formula.

10. The nutritional composition according to any one of claims 1 to 9, which is administered to an infant or young children, or elderly person.
